# EUROPEAN PATENT SPECIFICATION

(11) **EP 4 261 201 B1**
(45) Date of publication and mention of the grant of the patent: **12.08.2026**
(21) Application number: 21902558.2
(22) Date of filing: 06.12.2021
(51) Int. Cl.: C07C 227/18, C07C 229/64, C07C 303/28, C07C 309/66, C07C 309/73, C07C 227/42

(54) **METHOD FOR PREPARING 2-HYDROXY-5-[2-(4-(TRIFLUOROMETHYLPHENYL)ETHYLAMINO)]BENZOIC ACID**
VERFAHREN ZUR HERSTELLUNG VON 2-HYDROXY-5-[2-(4-(TRIFLUORMETHYLPHENYL)ETHYLAMINO)BENZOESÄURE
PROCÉDÉ DE PRÉPARATION D'ACIDE 2-HYDROXY-5-[2-(4-(TRIFLUOROMÉTHYLPHÉNYL)ÉTHYLAMINO)]BENZOÏQUE

(30) Priority: 08.12.2020 CN 202011445339
(43) Date of publication of application: 18.10.2023
(73) Proprietor: GNT Pharma Co., Ltd, Yongin-si, Gyeonggi-do, 17096 (KR)
(72) Inventor: ZHUANG, Chenghan, Zhejiang 322118 (CN); WANG, Lei, Zhejiang 322118 (CN); LU, Xin, Zhejiang 322118 (CN); ZHANG, Bo, Zhejiang 322118 (CN); GWAG, Byoung Joo, Yongin-si, Gyeonggi-do 17096 (KR); AHN, Chun San, Yongin-si, Gyeonggi-do 17096 (KR); JIN, Jing Yu, Yongin-si, Gyeonggi-do 17096 (KR); XU, Xinliang, Zhejiang 322118 (CN)
(74) Representative: Ipsilon
(86) International application number: PCT/CN2021/135804
(87) International publication number: WO 2022/121853

(56) References cited:
- WO-A2-2009/064084
- CN-A- 104 817 465
- CN-A- 112 409 201
- US-A1- 2007 298 129
- LEE IKCHOON ET AL: "Kinetics and mechanism of reactions between 2-phenylethyl benzenesulphonates and anilines in methanol", no. 8, 1 January 1988 (1988-01-01), GB, pages 1537 - 1540, XP093220331, ISSN: 0300-9580, Retrieved from the Internet <URL:https://pubs.rsc.org/en/content/articlepdf/1988/p2/p29880001537> DOI: 10.1039/p29880001537
- WU YU-LIANG, XIN LU, XIAO-LI CHEN, ZHANG XING-XIAN: "The synthesis of anti-Alzheimer's disease drug 2-hydroxy-5-2-n4-trifluoro methyl-phenyld-ethylamino -benzoic acid", CHINESE JOURNAL OF NEW DRUGS, GAI-KAN BIANJIBU, BEIJING, CN, vol. 21, no. 16, 31 December 2012 (2012-12-31), CN , pages 1930 - 1932, XP055941288, ISSN: 1003-3734
- AGGARWAL RANJANA, KUMAR RAJIV, KUMAR VINOD: "A facile and rapid one-pot synthesis of 1, 4-diaryl-2-mercaptoimidazoles under solvent-free conditions", JOURNAL OF SULFUR CHEMISTRY, vol. 28, no. 6, 31 December 2007 (2007-12-31), GB , pages 617 - 623, XP009537577, ISSN: 1741-5993, DOI: 10.1080/17415990701625035
- BAKTHADOSS MANICKAM, SRINIVASAN JAYAKUMAR, SELVAKUMAR RAMAN: "A Simple and Direct Synthesis of 3-Methylene-1, 4-diarylazetidin-2-ones and (E)-3-Arylidene-1-phenylazetidin-2-ones Using Baylis–Hillman Derivatives", AUSTRALIAN JOURNAL OF CHEMISTRY, C S I R O PUBLISHING, AU, vol. 67, no. 2, 1 January 2014 (2014-01-01), AU , pages 295, XP055941291, ISSN: 0004-9425, DOI: 10.1071/CH13382

## Description

### CROSS REFERENCE TO RELATED APPLICATIONS

This application claims the priority of Chinese Invention Patent Application No. 202011445339.4, filed with the China National Intellectual Property Administration on December 8, 2020, and titled with "METHOD FOR PREPARING 2-HYDROXY-5-[2-(4-(TRIFLUOROMETHYLPHENYL)ETHYLAMINO)]BENZOIC ACID"..

### FIELD OF THE INVENTION

The present disclosure relates to the field of drug synthesis methods, and relates to the synthesis of an anti-Alzheimer's disease drug, in particular to a method for preparing 2-hydroxy-5-[2-(4-(trifluoromethylphenyl)ethylamino)]benzoic acid.

### BACKGROUND OF THE INVENTION

2-hydroxy-5-[2-(4-(trifluoromethylphenyl)ethylamino)]benzoic acid is a novel cell necrosis inhibitor developed by GNT Pharma Co., Ltd., Korea, which can effectively treat neurological diseases such as Alzheimer's disease and Parkinson's syndrome, and has been well evaluated in clinical practice.

At present, there are few reports on the synthesis methods of this compound, and the processes involved are complex, the raw materials are expensive, and the practicality is not strong. Wu Yuliang et al. reported an industrially scalable synthetic route (Wu Yuliang, Lu Xin et al., Synthesis of an anti-Alzheimer's disease drug 2-hydroxy-5-[2-(4-(trifluoromethylphenyl)ethylamino)]benzoic acid [J], Chinese Journal of New Drugs, 2012, 21 (16): 1930-1932), which was achieved by condensation and hydrolysis of 2-(4-trifluoromethyl)phenethyl methanesulfonate (compound **4)** with methyl 5-aminosalicylate (compound **6)** to prepare the target product, which was applied in large-scale production.

However, the biggest problem with this route is that 2-(4-trifluoromethyl)phenethyl methanesulfonate has poor stability, low melting point (27-28°C), and is not easy to refine and purify. In particular, the meta-isomer impurity 2-(3-trifluoromethyl)phenethyl methanesulfonate is difficult to remove, and the content of the disubstituted impurity methyl 2-hydroxy-5-[N,N-bis(2-(4-(trifluoromethyl)phenyl)ethyl)amino]benzoate from the condensation reaction is high, and its hydrolysate will be brought into the final product, thereby affecting the quality of the final product. As the current quality control standards for APIs (Active Pharmaceutical Ingredient) are getting higher, there are many problems with synthesis via 2-(4-trifluoromethyl)phenethyl methanesulfonate.

For the problems existing in the current process, it is desired to find a suitable intermediate to replace 2-(4-trifluoromethyl)phenethyl methanesulfonate in order to solve the above problems and obtain high quality products.

### SUMMARY OF THE INVENTION

### TECHNICAL PROBLEM

In order to overcome the above-mentioned defects, the present disclosure provides a method for preparing 2-hydroxy-5-[2-(4-(trifluoromethylphenyl)ethylamino)]benzoic acid with high yield and purity.

### TECHNICAL SOLUTION

Specifically, the present disclosure provides a method for preparing 2-hydroxy-5-[2-(4-(trifluoromethylphenyl)ethylamino)]benzoic acid, comprising steps of:
(1) subjecting Compound I to a protection reaction with a protective reagent to obtain Compound II, 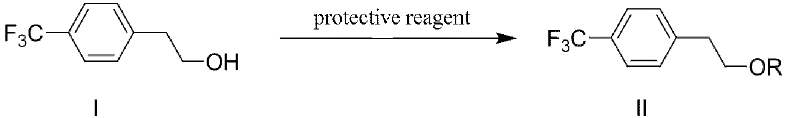
(2) subjecting Compound II to a condensation reaction with methyl 5-aminosalicylate to obtain Compound III, 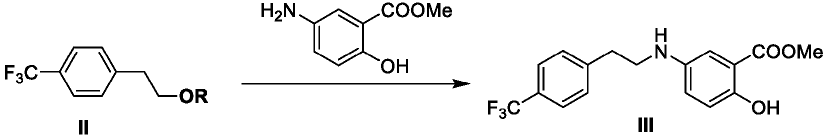
(3) subjecting Compound III to a hydrolysis reaction to obtain the target Compound IV, 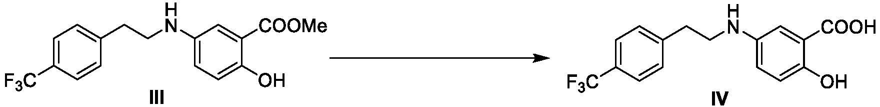 wherein, R is any one of p-methylbenzenesulfonyl, p-nitrobenzenesulfonyl, benzenesulfonyl, trifluoromethanesulfonyl and acetyl, preferably p-methylbenzenesulfonyl or benzenesulfonyl, more preferably p-methylbenzenesulfonyl.

In the above preparation method, the protection reaction is carried out at a temperature of 0-30°C, preferably 20-25°C.

In the above preparation method, the protection reaction is carried out in a solvent, which is at least one of toluene, ethylbenzene, xylene, methylene chloride and chloroform, preferably toluene.

Preferably, in the above preparation method, step (1) further comprises a step of refining the crude Compound II.

More preferably, in the above preparation method, the refining is done by crystallization, which is performed by dissolving at 40-80°C and insulation crystallizing at 0-40°C, preferably dissolving at 50-60°C and insulation crystallizing at 20-30°C; the solvent used for the crystallization is at least one of ethyl acetate, n-hexane, n-heptane, toluene, methylene chloride, methanol, ethanol, isopropanol and water, preferably n-heptane.

In the above preparation method, the condensation reaction is carried out at a temperature of 30-100°C, preferably 80-90°C.

In the above preparation method, the condensation reaction is carried out in a solvent, which is at least one of toluene, acetonitrile, N,N-dimethylformamide, dimethyl sulfoxide and tetrahydrofuran, preferably toluene.

In the above preparation method, the condensation reaction is carried out in the presence of a base, which is at least one of triethylamine, *N,N*-diisopropylethylamine, 1,8-diazabicyclo[5.4.0]undec-7-ene (DBU) and pyridine, preferably triethylamine.

Preferably, in the above preparation method, step (2) further comprises a step of forming a salt of the crude Compound III using an acid or an aqueous solution thereof.

More preferably, in the above preparation method, the salt-forming is performed by using an inorganic acid, which is any one of hydrochloric acid, sulfuric acid, nitric acid and phosphoric acid, preferably sulfuric acid.

In the above preparation method, the hydrolysis reaction is carried out at a temperature of 60-100°C, preferably 80-85°C.

In the above preparation method, the hydrolysis reaction is carried out in the presence of an acid, which is sulfuric acid.

In the above preparation method, the hydrolysis reaction is carried out with nitrogen bubbling.

### TECHNICAL EFFECTS

By using specific protective reagents for protecting the hydroxy group in Compound I (i.e., 2-(4-trifluoromethyl)phenethyl alcohol), combined with optimization of other process parameters, the method of the present disclosure is able to effectively remove the meta-isomer impurities in step (1) and the disubstituted impurities in step (2), thus obtaining 2-hydroxy-5-[2-(4-(trifluoromethylphenyl)ethylamino)]benzoic acid with high yield and purity, thereby satisfying the requirements of high quality standards for APIs in preparation research.

### DESCRIPTION OF DRAWINGS

Fig. 1 shows the HPLC chromatogram of the target product prepared in Example 1.
Fig. 2 shows the HPLC chromatogram of the target product prepared in Example 2.
Fig. 3 shows the HPLC chromatogram of the target product prepared in Example 3.
Fig. 4 shows the HPLC chromatogram of the target product prepared in Example 4.
Fig. 5 shows the HPLC chromatogram of the target product prepared in Comparative Example.

### DETAILED EMBODIMENTS

Unless otherwise specified, the term "compound" as used in the present disclosure includes all stereoisomeric forms, geometric isomeric forms, tautomeric forms and isotopically labeled forms of the compound.

Unless otherwise specified, the numerical range represented by "numerical value A - numerical value B" used in the present disclosure refers to a range comprising endpoint values A and B.

Unless otherwise specified, the numerical range represented by "above" or "below" used in the present disclosure refers to a numerical range including the number.

Hereinafter, the present disclosure will be described in detail.

The present disclosure provides a method for preparing 2-hydroxy-5-[2-(4-(trifluoromethylphenyl)ethylamino)]benzoic acid with high yield and purity.

The method comprises steps of:
(1) subjecting Compound I to a protection reaction with a protective reagent to obtain Compound II, 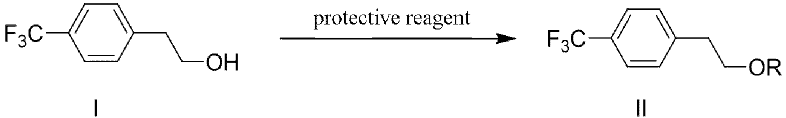
(2) subjecting Compound II to a condensation reaction with methyl 5-aminosalicylate to obtain Compound III, 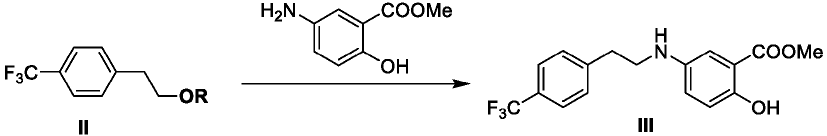
(3) subjecting Compound III to a hydrolysis reaction to obtain the target Compound IV, which is 2-hydroxy-5-[2-(4-(trifluoromethylphenyl)ethylamino)]benzoic acid, 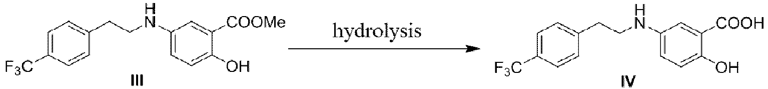 wherein, R is any one of p-methylbenzenesulfonyl, p-nitrobenzenesulfonyl, benzenesulfonyl, trifluoromethanesulfonyl and acetyl, preferably p-methylbenzenesulfonyl or benzenesulfonyl, more preferably p-methylbenzenesulfonyl.

### Step (1)

Step (1) is a step of subjecting Compound I (i.e., 2-(4-trifluoromethyl)phenethyl alcohol) to a protection reaction with a protective reagent to obtain Compound II (i.e., 2-(4-trifluoromethyl)phenethyl sulfonate or carboxylate).

In step (1), the selection of the protecive group plays a key role in the removal of the meta-isomer impurities in this step and the control of the disubstituted impurities in step (2).

In an embodiment of the present disclosure, the protective reagent used to protect Compound I in step (1) may be any one of p-methylbenzenesulfonyl chloride, trifluoromethanesulfonyl chloride, acetyl chloride, benzenesulfonyl chloride and p-nitrobenzenesulfonyl chloride, or any one of their corresponding anhydrides. Accordingly, the protecive group (i.e., group R) in Compound II may be any one of p-methylbenzenesulfonyl, trifluoromethanesulfonyl, acetyl, benzenesulfonyl and p-nitrophenylsulfonyl.

In a preferred embodiment of the present disclosure, the protecting reagent used to protect Compound I in step (1) may be p-methylbenzenesulfonyl chloride or benzenesulfonyl chloride.

In a more preferred embodiment of the present disclosure, the protecting reagent used to protect compound I in step (1) may be p-methylbenzenesulfonyl chloride.

The intermediate 2-(4-trifluoromethyl)phenethyl methanesulfonate used in the prior art has poor stability, low melting point, and is not easy to refine and purify. In particular, the isomer impurities are difficult to remove, and the content of the disubstituted impurities in the condensation reaction is high, thereby affecting the quality of the final product. In contrast, by using the above-mentioned protective reagents, the intermediates obtained by the present disclosure are 2-(4-trifluoromethyl)phenethyl p-methylbenzenesulfonate, 2-(4-trifluoromethyl)phenethyl trifluoromethanesulfonate, 2-(4-trifluoromethyl)phenethyl acetate, 2-(4-trifluoromethyl)phenethyl benzenesulfonate and 2-(4-trifluoromethyl)phenethyl p-nitrobenzenesulfonate, respectively, which overcome the above-mentioned shortcomings of 2-(4-trifluoromethyl)phenethyl methanesulfonate. As a result, a final product 2-hydroxy-5-[2-(4-(trifluoromethylphenyl)ethylamino)]benzoic acid can be prepared with high purity and yield.

In an embodiment of the present disclosure, the protection reaction in step (1) is carried out at low temperature.

In a preferred embodiment of the present disclosure, the protection reaction in step (1) is carried out at 0-30°C.

In a preferred embodiment of the present disclosure, the protection reaction in step (1) is carried out at 20-25°C.

In an embodiment of the present disclosure, the protection reaction in step (1) may be carried out in a solvent.

In a preferred embodiment of the present disclosure, the protection reaction in step (1) may be carried out in an organic solvent, which may be at least one of toluene, ethylbenzene, xylene, methylene chloride and chloroform.

In a preferred embodiment of the present disclosure, the protection reaction in step (1) may be carried out in toluene.

After the protection reaction in step (1) is completed, water may be added to the system, and stirred for layer separation, and then the separated organic phase may be concentrated under reduced pressure to obtain a crude Compound II, which is further refined in order to obtain a Compound II with high purity.

In an embodiment of the present disclosure, step (1) may further comprise a step of refining the crude Compound II. Generally, the refining step can be done by means of crystallization. And, the selection of the solvent used for the crystallization has a great influence on the removal of meta-isomers in step (1).

In a preferred embodiment of the present disclosure, the solvent used for the crystallization may be at least one of ethyl acetate, n-hexane, n-heptane, toluene, methylene chloride, methanol, ethanol, isopropanol and water.

In a more preferred embodiment of the present disclosure, the solvent used for the crystallization may be n-heptane.

In addition, when refining the crude Compound II by means of crystallization, the temperature of the crystallization can also be controlled to facilitate the removal of the impurities and the precipitation of the target product. If the temperature of the crystallization is too high, the yield will be affected, and if it is too low, the purity of Compound II will be reduced.

In an embodiment of the present disclosure, the crystallization may be performed by dissolving at 40-80°C and insulation crystallizing at 0-40°C.

In a preferred embodiment of the present disclosure, the crystallization may be performed by dissolving at 50-60°C and insulation crystallizing at 20-30°C.

### Step (2)

Step (2) is a step of subjecting Compound II to a condensation reaction with methyl 5-aminosalicylate to obtain Compound III (i.e., methyl 2-hydroxy-5-[2-(4-(trifluoromethylphenyl) ethylamino)]benzoate).

In step (2), the reaction temperature has a great influence on the disubstituted impurities, and an increase in temperature will lead to a significant increase in the content of the disubstituted impurities.

In an embodiment of the present disclosure, the condensation reaction in step (2) may be carried out at a temperature of 30-100°C.

In a preferred embodiment of the present disclosure, the condensation reaction in step (2) may be carried out at a temperature of 80-90°C.

In an embodiment of the present disclosure, the condensation reaction in step (2) may be carried out in a solvent.

In a preferred embodiment of the present disclosure, the condensation reaction in step (2) may be carried out in an organic solvent, which may be at least one of toluene, acetonitrile, *N,N-*dimethylformamide, dimethyl sulfoxide and tetrahydrofuran.

In a more preferred embodiment of the present disclosure, the condensation reaction in step (2) may be carried out in toluene.

In an embodiment of the present disclosure, the condensation reaction in step (2) may be carried out in the presence of a base, which may be at least one of triethylamine, *N,N-*diisopropylethylamine, 1,8-diazabicyclo[5.4.0]undec-7-ene (DBU) and pyridine.

In an embodiment of the present disclosure, the condensation reaction in step (2) may be carried out in the presence of triethylamine.

After the condensation reaction in step (2) is completed, water may be added to the system, and stirred for layer separation, and then the separated organic phase may be concentrated under reduced pressure to obtain a crude Compound III.

Since the crude Compound III is usually difficult to exist in solid form and cannot be directly crystallized, it needs to be converted into a salt form, which will be beneficial to obtain a solid, and achieve the purpose of separation and purification through crystallization.

In an embodiment of the present disclosure, step (2) further comprises a step of forming a salt of the crude Compound III using an acid (or a solution, preferably an aqueous solution thereof). Generally, the salt-forming step can be performed by using an inorganic or organic acid. Common inorganic acids include (but not limited to) hydrochloric acid, sulfuric acid, nitric acid, and phosphoric acid. Common organic acids include (but not limited to) acetic acid, lactic acid, citric acid, malic acid and tartaric acid.

In a preferred embodiment of the present disclosure, the acid used for the salt-forming may be any one of hydrochloric acid, sulfuric acid, nitric acid and phosphoric acid, or an aqueous solution of any concentration thereof.

In a more preferred embodiment of the present disclosure, the acid used for the salt-forming may be sulfuric acid, or an aqueous solution of any concentration thereof.

Accordingly, when the acid used for the salt-forming is sulfuric acid, the acid addition salt of Compound III is preferably a hemisulfate.

### Step (3)

Step (3) is a step of subjecting Compound III (i.e., methyl 2-hydroxy-5-[2-(4-(trifluoromethylphenyl)ethylamino)]benzoate) or an acid addition salt (such as hemisulfate) thereof to a hydrolysis reaction to obtain the target Compound IV (i.e., 2-hydroxy-5-[2-(4-(trifluoromethylphenyl)ethylamino)]benzoic acid).

In an embodiment of the present disclosure, the hydrolysis reaction in step (3) may be carried out at a temperature of 60-100°C.

In a preferred embodiment of the present disclosure, the hydrolysis reaction in step (3) may be carried out at a temperature of 80-85°C.

In an embodiment of the present disclosure, the hydrolysis reaction in step (3) may be carried out in the presence of acid, which may be sulfuric acid, or an aqueous solution of any concentration thereof.

In an embodiment of the present disclosure, the acid used for hydrolysis may be sulfuric acid.

In order to make the prepared Compound IV have higher purity, activated carbon may further be added for decolorization.

In an embodiment of the present disclosure, the hydrolysis reaction in step (3) may be carried out with nitrogen bubbling, which can obviously shorten the reaction time and improve the reaction conversion rate.

In addition, it should be particularly noted that, unless otherwise defined, each step in the preparation method of the present disclosure detects the endpoint of the reaction by HPLC method; the reaction raw materials are generally reacted according to the chemical reaction stoichiometry ratio or in excess; the amount of reaction solvent and/or catalyst can be adjusted according to the amount of the reaction raw materials, specifically, it can be increased with more reaction raw materials, and decreased with less reaction raw materials.

For a better understanding of the technical solutions of the present disclosure, it is further described below in connection with specific examples, but those of ordinary skill in the art should recognize that the present disclosure is not limited to these examples.

### Example 1:

### (1) Preparation of 2-(4-trifluoromethyl)phenethyl p-methylbenzenesulfonate:

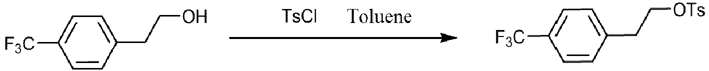

In a 500 mL volumetric flask, toluene (230 g) and p-methylbenzenesulfonyl chloride (100 g, 0.53 mol) were added, stirred to dissolve, and set aside. In a 1 L reaction flask, toluene (140 g), 40 wt% of sodium hydroxide aqueous solution (140 g, 1.4 mol), and 2-(4-trifluoromethyl)phenethyl alcohol (95 g, 0.5 mol) were added, and cooled down to 0-10°C. Then a toluene solution of p-methylbenzenesulfonyl chloride was added dropwise. Thereafter, it was reacted at 20-25°C for 6 hours and monitored by GC. After the reaction was completed, drinking water was added, and stirred for layer separation. The organic phase was concentrated under reduced pressure to obtain a concentrated crude product. The temperature was raised to 50-60°C, and n-heptane (170 g) was added dropwise. Thereafter, it was cooled down to 20-25°C, crystallized, suction filtered, and dried to obtain a dry product of the target compound (163 g), with a HPLC purity of 99.6%, a content of meta-isomer impurities of 0.03%, and a yield of 95%.

### (2) Preparation of methyl 2-hydroxy-5-[2-(4-(trifluoromethylphenyl)ethylamino)]benzoate hemisulfate:

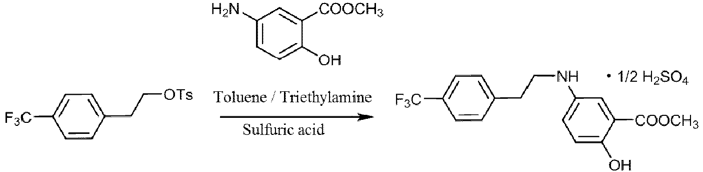

In a 1 L reaction flask, 2-(4-trifluoromethyl)phenethyl p-methylbenzenesulfonate (68 g, 0.20 mol), methyl 5-aminosalicylate (35 g, 0.21 mol), toluene (200 g), and triethylamine (24 g, 0.24 mol) were added. It was heated to and kept at 80-90°C, and reacted for 14 hours. The reaction was stopped after sampling and passing the test. Thereafter, drinking water was added, and stirred for layer separation. The organic phase was concentrated under reduced pressure until there was no distillate. Methanol (160 g) was added thereto, stirred to dissolve, and heated to 35-40°C, and 50 wt% of sulfuric acid aqueous solution (23 g, 0.12 mol) was added dropwise. Thereafter, it was stirred for 15 minutes, cooled down slowly to 25-30°C, insulation crystallized for 1 hour, suction filtered, and dried to obtain a hemisulfate of Compound III (67 g, 0.17 mol), with a HPLC purity of 99% and a yield of 86.3%.

### (3) Preparation of 2-hydroxy-5-[2-(4-(trifluoromethylphenyl)ethylamino)]benzoic acid:

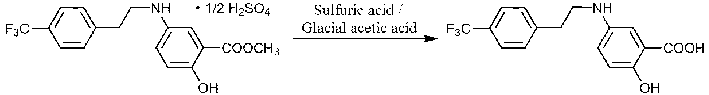

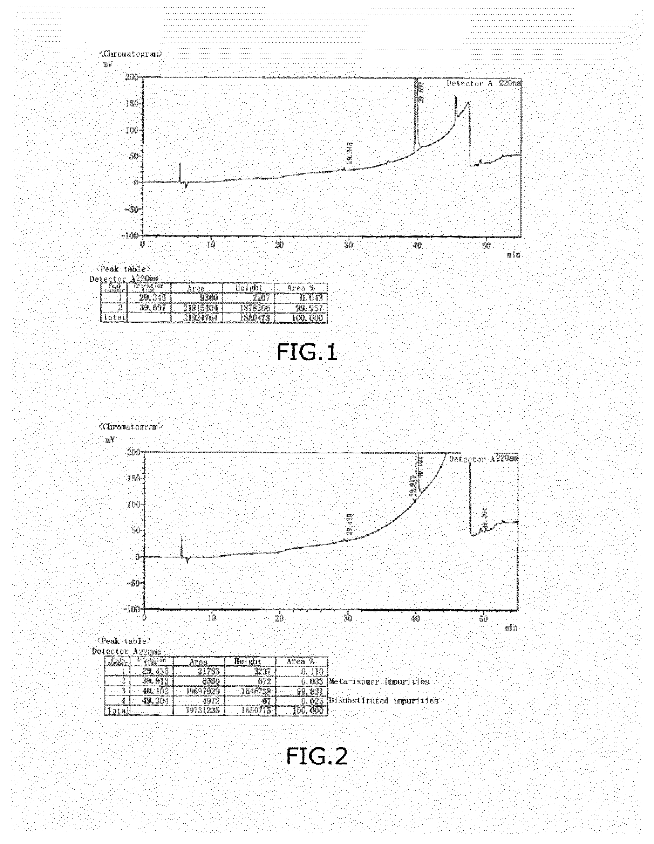

In a 500 mL reaction flask, 40 wt% of sulfuric acid aqueous solution (300 g), glacial acetic acid (100 g) and the hemisulfate of Compound III (77 g) were added. The temperature was raised to 80-85°C, while nitrogen was introduced into the reaction solution, and the bubbling reaction was carried out for 4 hours. After sampling and passing the test, activated carbon was added for decolorization, and filtered. The filtrate was slowly cooled down to -5 to 5°C, kept for 1 hour, suction filtered, and dried to obtain a dry product of Compound IV (58.4 g), with a HPLC purity of 99.957% and a yield of 93%. The disubstituted impurities and meta-isomer impurities were not detected (as shown in Fig. 1).

### Example 2:

### (1) Preparation of 2-(4-trifluoromethyl)phenethyl benzenesulfonate:

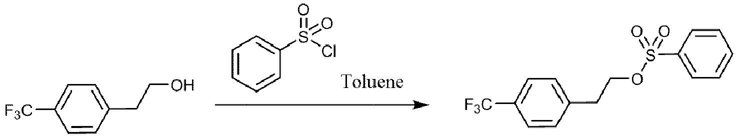

In a 500 mL volumetric flask, toluene (230 g) and benzenesulfonyl chloride (93 g, 0.53 mol) were added, stirred to dissolve, and set aside. In a 1 L reaction flask, toluene (140 g), 40 wt% of sodium hydroxide aqueous solution (140 g, 1.4 mol), and 2-(4-trifluoromethyl)phenethyl alcohol (95 g, 0.5 mol) were added, and cooled down to 0-10°C. Then a toluene solution of benzenesulfonyl chloride was added dropwise. Thereafter, it was reacted at 20-25°C for 8 hours and monitored by GC. After the reaction was completed, drinking water was added, and stirred for layer separation. The organic phase was concentrated under reduced pressure to obtain a concentrated crude product. The temperature was raised to 50-60°C, and a mixed solvent of ethyl acetate and n-heptane (150 g) was added dropwise. Thereafter, it was cooled down to 20-25°C, crystallized, suction filtered, and dried to obtain a dry product of the target compound (145 g, 0.44 mol), with a HPLC purity of 98% and a yield of 89%.

### (2) Preparation of methyl 2-hydroxy-5-[2-(4-(trifluoromethylphenyl)ethylamino)]benzoate hemisulfate:

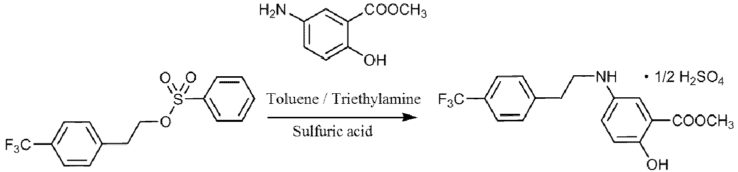

In a 1 L reaction flask, 2-(4-trifluoromethyl)phenethyl benzenesulfonate (66 g, 0.20 mol), methyl 5-aminosalicylate (35 g, 0.21 mol), toluene (200 g), and 24 g of triethylamine (24 g, 0.24 mol) were added. It was heated to and kept at 90-100°C, and reacted for 12 hours. The reaction was stopped after sampling and passing the test. Thereafter, drinking water was added, and stirred for layer separation. The organic phase was concentrated under reduced pressure until there was no distillate. Methanol (168 g) was added thereto, stirred to dissolve, and heated to 35-40°C, and 50 wt% of sulfuric acid aqueous solution (23 g, 0.12 mol) was added dropwise. Thereafter, it was stirred for 15 minutes, cooled down slowly to 25-30°C, insulation crystallized for 1 hour, suction filtered, and dried to obtain a hemisulfate of Compound III (62 g), with a HPLC purity of 98.5% and a yield of 80%.

### (3) Preparation of 2-hydroxy-5-[2-(4-(trifluoromethylphenyl)ethylamino)]benzoic acid:

In a 500 mL reaction flask, 40 wt% of sulfuric acid aqueous solution (240 g), glacial acetic acid (80 g) and the hemisulfate of Compound III (60 g) were added. The temperature was raised to 80-85°C, while nitrogen was introduced into the reaction solution, and the bubbling reaction was carried out for 4 hours. After sampling and passing the test, activated carbon was added for decolorization, and filtered. The filtrate was slowly cooled down to -5 to 5°C, kept for 1 hour, suction filtered, and dried to obtain a dry product of Compound IV (44.5 g), with a HPLC purity of 99.831%, a content of disubstituted impurities of 0.025%, a content of meta-isomer impurities of 0.033% (as shown in Fig. 2), and a yield of 91%.

### Example 3:

### (1) Preparation of 2-(4-trifluoromethyl)phenethyl acetate:

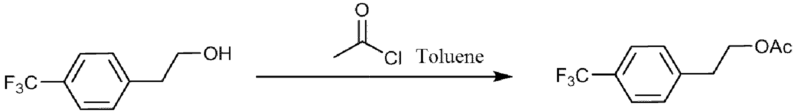

In a 500 mL volumetric flask, toluene (230 g) and acetyl chloride (41.6 g, 0.53 mol) were added, stirred to dissolve, and set aside. In a 1 L reaction flask, toluene (140 g), 40 wt% of sodium hydroxide aqueous solution (140 g, 1.4 mol), and 2-(4-trifluoromethyl)phenethyl alcohol (95 g, 0.5 mol) were added, and cooled down to 0-10°C. Then a toluene solution of acetyl chloride was added dropwise. Thereafter, it was reacted at 20-25°C for 8 hours and monitored by GC. After the reaction was completed, drinking water was added, and stirred for layer separation. The organic phase was concentrated under reduced pressure to obtain a concentrated crude product. The temperature was raised to 50-60°C, and a mixed solvent of ethyl acetate and n-heptane (150 g) was added dropwise. Thereafter, it was cooled down to 20-25°C, crystallized, suction filtered, and dried to obtain a dry product of the target compound (93 g, 0.4 mol), with a HPLC purity of 98% and a yield of 80%.

### (2) Preparation of methyl 2-hydroxy-5-[2-(4-(trifluoromethylphenyl)ethylamino)]benzoate hemisulfate:

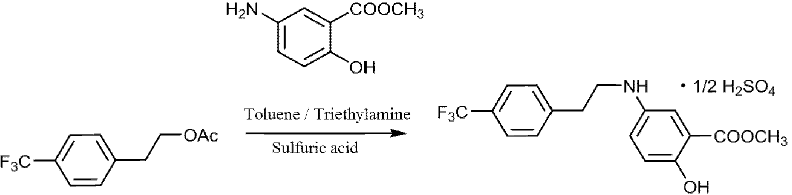

In a 1 L reaction flask, 2-(4-trifluoromethyl)phenethyl acetate (46.4 g, 0.20 mol), methyl 5-aminosalicylate (35 g, 0.21 mol), toluene (200 g), and 24 g of triethylamine (24 g, 0.24 mol) were added. It was heated to and kept at 90-100°C, and reacted for 12 hours. The reaction was stopped after sampling and passing the test. Thereafter, drinking water was added, and stirred for layer separation. The organic phase was concentrated under reduced pressure until there was no distillate. Methanol (168 g) was added thereto, stirred to dissolve, and heated to 35-40°C, and 50 wt% of sulfuric acid aqueous solution (23 g, 0.12 mol) was added dropwise. Thereafter, it was stirred for 15 minutes, cooled down slowly to 25-30°C, insulation crystallized for 1 hour, suction filtered, and dried to obtain a hemisulfate of Compound III (60 g, 0.15 mol), with a HPLC purity of 98.5% and a yield of 77%.

### (3) Preparation of 2-hydroxy-5-[2-(4-(trifluoromethylphenyl)ethylamino)]benzoic acid:

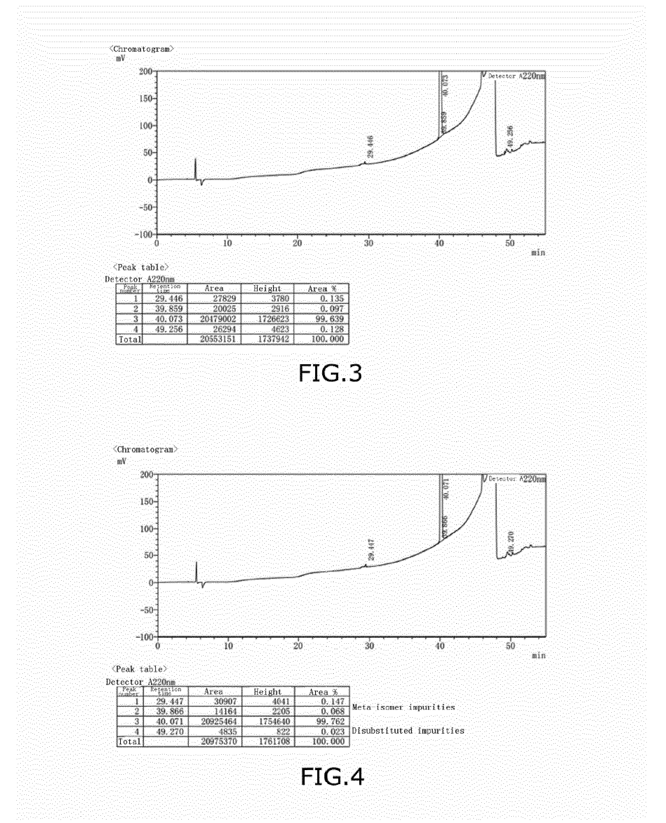

In a 500 mL reaction flask, 40 wt% of sulfuric acid aqueous solution (240 g), glacial acetic acid (80 g) and the hemisulfate of Compound III (60 g) were added. The temperature was raised to 80-85°C, while nitrogen was introduced into the reaction solution, and the bubbling reaction was carried out for 4 hours. After sampling and passing the test, activated carbon was added for decolorization, and filtered. The filtrate was slowly cooled down to -5 to 5°C, kept for 1 hour, suction filtered, and dried to obtain a dry product of Compound IV (40 g), with a HPLC purity of 99.639%, a content of disubstituted impurities of 0.128%, a content of meta-isomer impurities of 0.097% (as shown in Fig. 3), and a yield of 82%.

### Example 4 :

### (1) Preparation of 2-(4-trifluoromethyl)phenethyl trifluoromethanesulfonate:

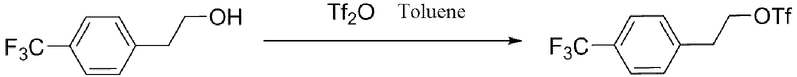

In a 500 mL volumetric flask, toluene (230 g) and trifluoromethanesulfonic anhydride (150 g, 0.53 mol) were added, stirred to dissolve, and set aside. In a 1 L reaction flask, toluene (140 g), triethylamine (64 g, 0.63 mol), and 2-(4-trifluoromethyl)phenethyl alcohol (95 g, 0.5 mol) were added, and cooled down to 0-10°C. Then a toluene solution of trifluoromethanesulfonic anhydride was added dropwise. Thereafter, it was reacted at 20-25°C for 8 hours and monitored by GC. After the reaction was completed, drinking water was added, and stirred for layer separation. The organic phase was concentrated under reduced pressure to obtain a concentrated crude product. The temperature was raised to 50-60°C, and a mixed solvent of ethyl acetate and n-heptane (150 g) was added dropwise. Thereafter, it was cooled down to 20-25°C, crystallized, suction filtered, and dried to obtain a dry product of the target compound (129 g, 0.4 mol), with a HPLC purity of 98.5% and a yield of 80%.

### (2) Preparation of methyl 2-hydroxy-5-[2-(4-(trifluoromethylphenyl)ethylamino)]benzoate hemisulfate:

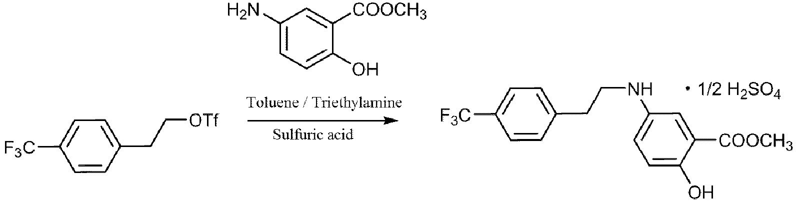

In a 1 L reaction flask, 2-(4-trifluoromethyl)phenethyl trifluoromethanesulfonate (64.5 g, 0.20 mol), methyl 5-aminosalicylate (35 g, 0.21 mol), toluene (200 g), and 24 g of triethylamine (24 g, 0.24 mol) were added. It was heated to and kept at 90-100°C, and reacted for 12 hours. The reaction was stopped after sampling and passing the test. Thereafter, drinking water was added, and stirred for layer separation. The organic phase was concentrated under reduced pressure until there was no distillate. Methanol (168 g) was added thereto, stirred to dissolve, and heated to 35-40°C, and 50 wt% of sulfuric acid aqueous solution (23 g, 0.12 mol) was added dropwise. Thereafter, it was stirred for 15 minutes, cooled down slowly to 25-30°C, insulation crystallized for 1 hour, suction filtered, and dried to obtain a hemisulfate of Compound III (61 g), with a HPLC purity of 98.5% and a yield of 78.3%.

### (3) Preparation of 2-hydroxy-5-[2-(4-(trifluoromethylphenyl)ethylamino)]benzoic acid:

In a 500 mL reaction flask, 40 wt% of sulfuric acid aqueous solution (240 g), glacial acetic acid (80 g) and the hemisulfate of Compound III (60 g) were added. The temperature was raised to 80-85°C, while nitrogen was introduced into the reaction solution, and the bubbling reaction was carried out for 4 hours. After sampling and passing the test, activated carbon was added for decolorization, and filtered. The filtrate was slowly cooled down to -5 to 5°C, kept for 1 hour, suction filtered, and dried to obtain a dry product of Compound IV (40.5 g), with a HPLC purity of 99.762%, a content of disubstituted impurities of 0.023%, a content of meta-isomer impurities of 0.068% (as shown in Fig. 4), and a yield of 83%.

### Comparative Example:

### (1) Preparation of 2-(4-trifluoromethyl)phenethyl methylsulfonate:

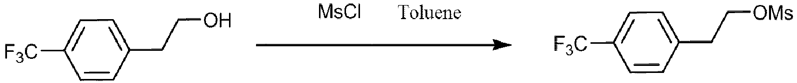

In a 500 mL volumetric flask, toluene (230 g) and methylsulfonyl chloride (60 g, 0.53 mol) were added, stirred to dissolve, and set aside. In a 1 L reaction flask, toluene (140 g), 40 wt% of sodium hydroxide aqueous solution (140 g, 1.4 mol), and 2-(4-trifluoromethyl)phenethyl alcohol (95 g, 0.5 mol) were added, and cooled down to 0-10°C. Then a toluene solution of methylsulfonyl chloride was added dropwise. Thereafter, it was reacted at 20-25°C for 10 hours and monitored by GC. After the reaction was completed, drinking water was added, and stirred for layer separation. The organic phase was concentrated under reduced pressure, then n-hexane (200 g) was added. Thereafter, it was cooled down to -10 to 0°C, crystallized, suction filtered, and dried under vacuum at 0-10°C to obtain a dry product of the target compound (116 g, 0.43 mol), with a HPLC purity of 97%, a content of meta-isomers of 0.3%, and a yield of 86%.

### (2) Preparation of methyl 2-hydroxy-5-[2-(4-(trifluoromethylphenyl)ethylamino)]benzoate hemisulfate:

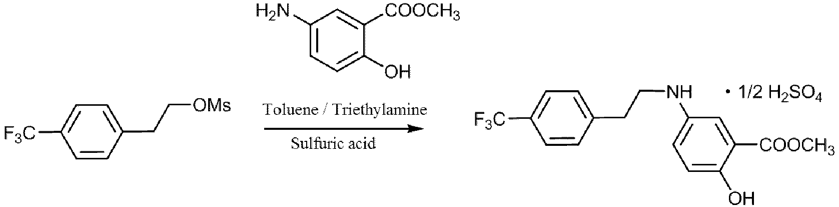

In a 1 L reaction flask, 2-(4-trifluoromethyl)phenethyl methylsulfonate (53.6 g, 0.20 mol), methyl 5-aminosalicylate (35 g, 0.21 mol), toluene (200 g), and triethylamine (24 g, 0.24 mol) were added. It was heated to and kept at 80-90°C, and reacted for 18 hours. The reaction was stopped after sampling and passing the test. Thereafter, drinking water was added, and stirred for layer separation. The organic phase was concentrated under reduced pressure until there was no distillate. Methanol (160 g) was added thereto, stirred to dissolve, and heated to 35-40°C, and 50 wt% of sulfuric acid aqueous solution (23 g, 0.12 mol) was added dropwise. Thereafter, it was stirred for 15 minutes, cooled down slowly to 25-30°C, insulation crystallized for 1 hour, suction filtered, and dried to obtain a hemisulfate of Compound III (57 g), with a HPLC purity of 97.3%, a content of meta-isomers of 0.24%, a content of disubstituted impurities of 1.8%, and a yield of 73%.

### (3) Preparation of 2-hydroxy-5-[2-(4-(trifluoromethylphenyl)ethylamino)]benzoic acid:

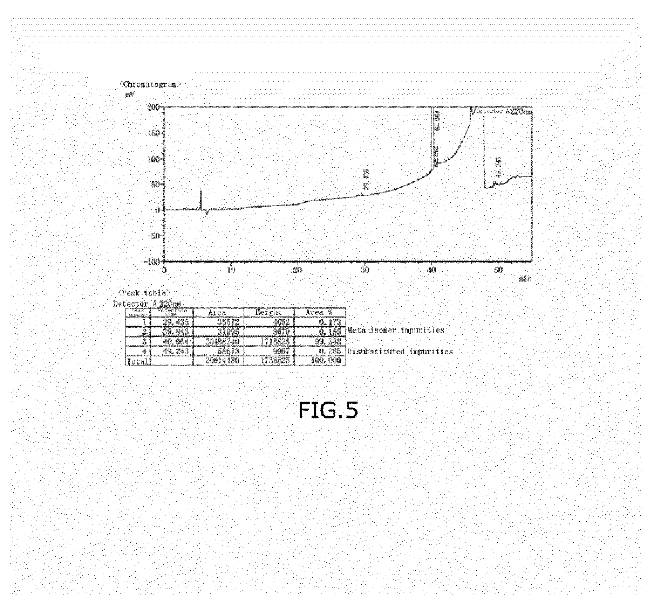

In a 500 mL reaction flask, 40% of sulfuric acid aqueous solution (300 g), glacial acetic acid (100 g) and Compound III (77 g) were added. The temperature was raised to 80-95°C and kept for 24 hours. After sampling and passing the test, activated carbon was added for decolorization, and filtered. The filtrate was slowly cooled down to -5 to 5°C, kept for 1 hour, suction filtered, and dried to obtain a dry product of Compound IV (52 g), with a HPLC purity of 99.388%, a content of disubstituted impurities of 0.285%, a content of meta-isomer impurities of 0.155% (as shown in Fig. 5), and a yield of 81%.

In the above Example 1, by using p-methylbenzenesulfonyl chloride as a protective reagent (i.e., using p-methylbenzenesulfonyl as a protecive group), the disubstituted impurities and meta-isomer impurities in the final product were not detected, and thus 2-hydroxy-5-[2-(4-(trifluoromethylphenyl)ethylamino)]benzoic acid was obtained with a very high purity of 99.957% and a high yield of 93%.

In the above Example 2, by using benzenesulfonyl chloride as a protective reagent (i.e., using benzenesulfonyl as a protecive group), disubstituted impurities and meta-isomer impurities with a content only less than 0.04% were detected in the final product, and thus 2-hydroxy-5-[2-(4-(trifluoromethylphenyl)ethylamino)]benzoic acid was obtained with a high purity of 99.831% and a yield of 91%.

In the above Example 3, by using acetyl chloride as a protective reagent (i.e., using acetyl as a protecive group), disubstituted impurities and meta-isomer impurities with a content of only less than 0.13% were detected in the final product, and thus 2-hydroxy-5-[2-(4-(trifluoromethylphenyl)ethylamino)]benzoic acid was obtained with a high purity of 99.639% and a yield of 82%.

In the above Example 4, by using trifluoromethanesulfonic anhydride as a protective reagent (i.e., using trifluoromethanesulfonyl as a protecive group), disubstituted impurities and meta-isomer impurities with a content of only less than 0.07% were detected in the final product, and thus 2-hydroxy-5-[2-(4-(trifluoromethylphenyl)ethylamino)]benzoic acid was obtained with a high purity of 99.762% and a yield of 83%.

However, in the Comparative Example, due to the use of methylsulfonyl chloride as a protective reagent, an intermediate 2-(4-trifluoromethyl)phenethyl methylsulfonate with poor stability was formed, resulting in a final product with a content of disubstituted impurities of 0.285%, a content of meta-isomer impurities of 0.155%, a purity of only 99.388%, and a yield of only 81%.

As can be seen from the above comparison, by using specific protective reagents, the present disclosure effectively controls the isomer impurities and disubstituted impurities of the final product 2-hydroxy-5-[2-(4-(trifluoromethylphenyl)ethylamino)]benzoic acid, the product quality is significantly improved, and the reaction yield is also greatly improved.

## Claims

1. A method for preparing 2-hydroxy-5-[2-(4-(trifluoromethylphenyl)ethylamino)]benzoic acid, comprising steps of:
1) subjecting Compound I to a protection reaction with a protective reagent to obtain Compound II, 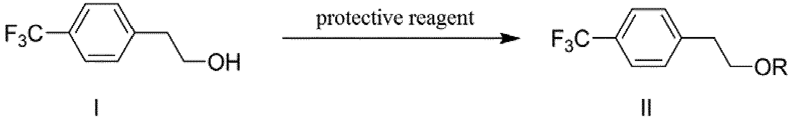
2) subjecting Compound II to a condensation reaction with methyl 5-aminosalicylate to obtain Compound III, 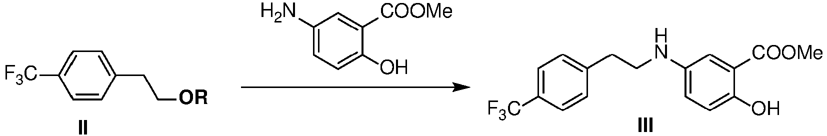
3) subjecting Compound III to a hydrolysis reaction to obtain the target Compound IV, 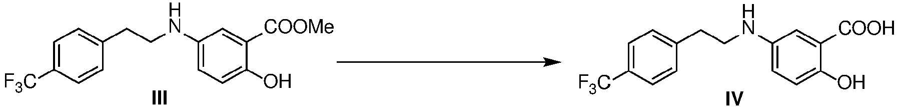 wherein, R is any one of p-methylbenzenesulfonyl, p-nitrobenzenesulfonyl, benzenesulfonyl, trifluoromethanesulfonyl and acetyl, preferably p-methylbenzenesulfonyl or benzenesulfonyl, more preferably p-methylbenzenesulfonyl.

2. The method according to claim 1, **characterized in that**,
the protection reaction is carried out at a temperature of 0-30°C, preferably 20-25°C.

3. The method according to claim 1, **characterized in that**,
the protection reaction is carried out in a solvent, which is at least one of toluene, ethylbenzene, xylene, methylene chloride and chloroform, preferably toluene.

4. The method according to claim 1, **characterized in that**,
step 1) further comprises a step of refining the crude Compound II.

5. The method according to claim 4, **characterized in that**,
the refining is done by crystallization, which is performed by dissolving at 40-80°C and insulation crystallizing at 0-40°C, preferably dissolving at 50-60°C and insulation crystallizing at 20-30°C;
the solvent used for the crystallization is at least one of ethyl acetate, n-hexane, n-heptane, toluene, methylene chloride, methanol, ethanol, isopropanol and water, preferably n-heptane.

6. The method according to claim 1, **characterized in that**,
the condensation reaction is carried out at a temperature of 30-100°C, preferably 80-90°C.

7. The method according to claim 1, wherein,
the condensation reaction is carried out in a solvent, which is at least one of toluene, acetonitrile, N,N-dimethylformamide, dimethyl sulfoxide and tetrahydrofuran, preferably toluene.

8. The method according to claim 1, **characterized in that**,
the condensation reaction is carried out in the presence of a base, which is at least one of triethylamine, N,N-diisopropylethylamine, 1,8-diazabicyclo[5.4.0]undec-7-ene and pyridine, preferably triethylamine.

9. The method according to claim 1, **characterized in that**,
step 2) further comprises a step of forming a salt of the crude Compound III using an acid or an aqueous solution thereof.

10. The method according to claim 9, **characterized in that**,
the salt-forming is performed by using an inorganic acid, which is any one of hydrochloric acid, sulfuric acid, nitric acid and phosphoric acid, preferably sulfuric acid.

11. The method according to claim 1, **characterized in that**,
the hydrolysis reaction is carried out at a temperature of 60-100°C, preferably 80-85°C.

12. The method according to claim 1, **characterized in that**,
the hydrolysis reaction is carried out in the presence of an acid, which is sulfuric acid.

13. The method according to claim 1, **characterized in that**,
the hydrolysis reaction is carried out with nitrogen bubbling.

## Patentansprüche

1. Verfahren zur Herstellung von 2-Hydroxy-5-[2-(4-(trifluormethylphenyl)ethylamino)]benzoesäure, welches die folgenden Schritte umfasst:
1) das Durchführen einer Schutzreaktion von Verbindung I mit einem Schutzreagenz unter Erhalt von Verbindung II, 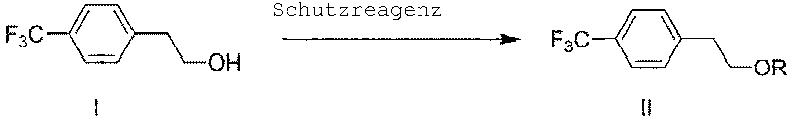
2) das Durchführen einer Kondensationsreaktion von Verbindung II mit 5-Aminosalicylsäuremethylester unter Erhalt von Verbindung III, 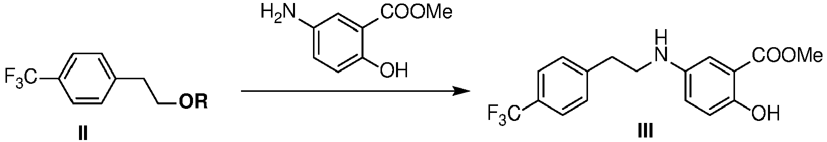
3) das Durchführen einer Hydrolysereaktion von Verbindung III unter Erhalt der Zielverbindung IV, 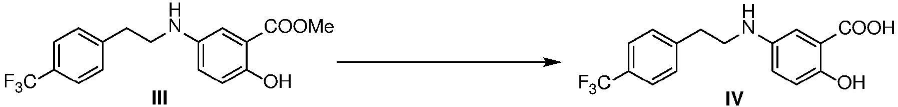 wobei R für p-Methylbenzolsulfonyl, p-Nitrobenzolsulfonyl, Benzolsulfonyl, Trifluormethansulfonyl oder Acetyl, vorzugsweise p-Methylbenzolsulfonyl oder Benzolsulfonyl, besonders bevorzugt p-Methylbenzolsulfonyl steht.

2. Verfahren nach Anspruch 1, **dadurch gekennzeichnet, dass**
die Schutzreaktion bei einer Temperatur von 0-30 °C, vorzugsweise 20-25 °C, durchgeführt wird.

3. Verfahren nach Anspruch 1, **dadurch gekennzeichnet, dass**
die Schutzreaktion in einem Lösungsmittel durchgeführt wird, bei dem es sich um mindestens eines von Toluol, Ethylbenzol, Xylol, Methylenchlorid und Chloroform, vorzugsweise Toluol, handelt.

4. Verfahren nach Anspruch 1, **dadurch gekennzeichnet, dass**
Schritt 1) weiterhin einen Schritt der Aufreinigung der rohen Verbindung II umfasst.

5. Verfahren nach Anspruch 4, **dadurch gekennzeichnet, dass**
die Aufreinigung durch Kristallisation erfolgt, die durch Lösen bei 40-80 °C und Isolationskristallisieren bei 0-40 °C, vorzugsweise Lösen bei 50-60 °C und Isolationskristallisieren bei 20-30 °C, durchgeführt wird,
wobei als Lösungsmittel für die Kristallisation wird mindestens eines von Essigsäureethylester, n-Hexan, n-Heptan, Toluol, Methylenchlorid, Methanol, Ethanol, Isopropanol und Wasser, vorzugsweise n-Heptan, verwendet wird.

6. Verfahren nach Anspruch 1, **dadurch gekennzeichnet, dass**
die Kondensationsreaktion bei einer Temperatur von 30-100 °C, vorzugsweise 80-90 °C, durchgeführt wird.

7. Verfahren nach Anspruch 1, wobei
die Kondensationsreaktion in einem Lösungsmittel durchgeführt wird, bei dem es sich um mindestens eines von Toluol, Acetonitril, N,N-Dimethylformamid, Dimethylsulfoxid und Tetrahydrofuran, vorzugsweise Toluol, handelt.

8. Verfahren nach Anspruch 1, **dadurch gekennzeichnet, dass**
die Kondensationsreaktion in Gegenwart einer Base durchgeführt wird, bei der es sich um mindestens eine von Triethylamin, N,N-Diisopropylethylamin, 1,8-Diazabicyclo[5.4.0]undec-7-en und Pyridin, vorzugsweise Triethylamin, handelt.

9. Verfahren nach Anspruch 1, **dadurch gekennzeichnet, dass**
Schritt 2) weiterhin einen Schritt des Bildens eines Salzes der rohen Verbindung III unter Verwendung einer Säure oder einer wässrigen Lösung davon umfasst.

10. Verfahren nach Anspruch 9, **dadurch gekennzeichnet, dass**
die Salzbildung unter Verwendung einer anorganischen Säure erfolgt, bei der es sich um Salzsäure, Schwefelsäure, Salpetersäure oder Phosphorsäure, vorzugsweise Schwefelsäure, handelt.

11. Verfahren nach Anspruch 1, **dadurch gekennzeichnet, dass**
die Hydrolysereaktion bei einer Temperatur von 60-100 °C, vorzugsweise 80-85 °C, durchgeführt wird.

12. Verfahren nach Anspruch 1, **dadurch gekennzeichnet, dass**
die Hydrolysereaktion in Gegenwart einer Säure, bei der es sich um Schwefelsäure handelt, durchgeführt wird.

13. Verfahren nach Anspruch 1, **dadurch gekennzeichnet, dass**
die Hydrolysereaktion unter Einleiten von Stickstoff durchgeführt wird.

## Revendications

1. Procédé de préparation d'acide 2-hydroxy-5-[2-(4-(trifluorométhylphényl)éthylamino)]benzoïque, comprenant les étapes consistant à :
1) soumettre le composé I à une réaction de protection avec un réactif protecteur pour obtenir le composé II, 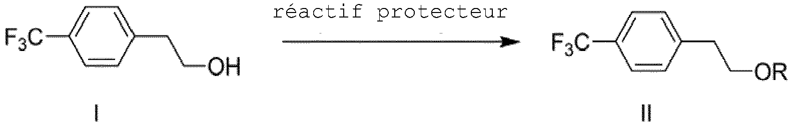
2) soumettre le composé II à une réaction de condensation avec du 5-aminosalicylate de méthyle pour obtenir le composé III, 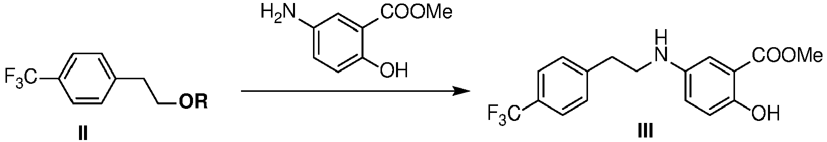
3) soumettre le composé III à une réaction d'hydrolyse pour obtenir le composé IV cible, 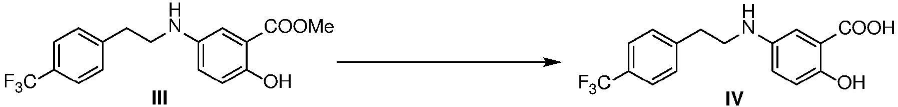 dans lequel, R est l'un quelconque des groupes p-méthylbenzènesulfonyle, p-nitrobenzènesulfonyle, benzènesulfonyle, trifluorométhanesulfonyle et acétyle, de préférence p-méthylbenzènesulfonyle ou benzènesulfonyle, plus préférablement p-méthylbenzènesulfonyle.

2. Procédé selon la revendication 1, **caractérisé en ce que**,
la réaction de protection est mise en œuvre à une température de 0 à 30 °C, de préférence de 20 à 25 °C.

3. Procédé selon la revendication 1, **caractérisé en ce que**,
la réaction de protection est mise en œuvre dans un solvant, qui est au moins l'un du toluène, de l'éthylbenzène, du xylène, du chlorure de méthylène et du chloroforme, de préférence le toluène.

4. Procédé selon la revendication 1, **caractérisé en ce que**,
l'étape 1) comprend en outre une étape de raffinage du composé II brut.

5. Procédé selon la revendication 4, **caractérisé en ce que**,
le raffinage est réalisé par cristallisation, qui est effectuée par dissolution à 40 à 80 °C et cristallisation isotherme à 0 à 40 °C, de préférence dissolution à 50 à 60 °C et cristallisation isotherme à 20 à 30 °C ;
le solvant utilisé pour la cristallisation est au moins l'un de l'acétate d'éthyle, du n-hexane, du n-heptane, du toluène, du chlorure de méthylène, du méthanol, de l'éthanol, de l'isopropanol et de l'eau, de préférence le n-heptane.

6. Procédé selon la revendication 1, **caractérisé en ce que**,
la réaction de condensation est mise en œuvre à une température de 30 à 100 °C, de préférence de 80 à 90 °C.

7. Procédé selon la revendication 1, dans lequel,
la réaction de condensation est mise en œuvre dans un solvant, qui est au moins l'un du toluène, de l'acétonitrile, du *N,N*-diméthylformamide, du diméthylsulfoxyde et du tétrahydrofurane, de préférence le toluène.

8. Procédé selon la revendication 1, **caractérisé en ce que**,
la réaction de condensation est mise en œuvre en présence d'une base, qui est au moins l'une de la triéthylamine, de la *N,N*-diisopropyléthylamine, du 1,8-diazabicyclo[5.4.0]undéc-7-ène et de la pyridine, de préférence la triéthylamine.

9. Procédé selon la revendication 1, **caractérisé en ce que**,
l'étape 2) comprend en outre une étape consistant à former un sel du composé III brut à l'aide d'un acide ou d'une solution aqueuse de celui-ci.

10. Procédé selon la revendication 9, **caractérisé en ce que**,
la formation de sel est effectuée à l'aide d'un acide inorganique, qui est l'un quelconque de l'acide chlorhydrique, de l'acide sulfurique, de l'acide nitrique et de l'acide phosphorique, de préférence l'acide sulfurique.

11. Procédé selon la revendication 1, **caractérisé en ce que**,
la réaction d'hydrolyse est mise en œuvre à une température de 60 à 100 °C, de préférence de 80 à 85 °C.

12. Procédé selon la revendication 1, **caractérisé en ce que**,
la réaction d'hydrolyse est mise en œuvre en présence d'un acide, qui est l'acide sulfurique.

13. Procédé selon la revendication 1, **caractérisé en ce que**,
la réaction d'hydrolyse est mise en œuvre sous barbotage d'azote.
